# EUROPÄISCHE PATENTANMELDUNG

(11) **EP 0 771 784 A1**
(43) Veröffentlichungstag der Anmeldung: **07.05.1997**
(21) Anmeldenummer: 96115705.4
(22) Anmeldetag: 01.10.1996
(51) Int. Cl.: C07C 209/26, C07C 209/48, C07C 211/35, B01J 25/00

(54) **Verfahren zur Herstellung von 3-Aminomethyl-3,5,5-trimethylcyclohexylamin**

(30) Priorität: 30.10.1995 DE 19540191
(71) Anmelder: Degussa Aktiengesellschaft, 60311 Frankfurt (DE)
(72) Erfinder: Haas, Thomas, Dr., 60316 Frankfurt (DE); Burmeister, Roland, Dr., 63826 Geiselbach (DE); Arntz, Dietrich, Dr., 61440 Oberursel (DE); Weber, Karl-Ludwig, Dr., 64807 Dieburg (DE); Berweiler, Monika, 63477 Maintal (DE)

(57) **Zusammenfassung**

Die Erfindung betrifft ein verbessertes Verfahren zur Herstellung von 3-Aminomethyl-3,5,5-trimethylcyclohexylamin (=Isophorondiamin) aus Isophoronnitril.

Es ist bekannt, zur Herstellung von Isophorondiamin Isophoronnitril in einer ersten Stufe zu iminieren und das Reaktionsgemisch in einer zweiten Stufe in Gegenwart eines Festbett-Hydrierkatalysators auf der Basis von Kobalt nach Raney aminierend zu hydrieren.

Durch erfindungsgemäße Verwendung eines in spezieller Weise hergestellten Festbett-Hydrierkatalysators - Mischen einer pulverförmigen Co-haltigen Legierung nach Raney mit pulverförmigem Kobalt, Sintern des Pulvergemischs zu Formlingen und Aktivieren derselben durch Auslaugen mittels Alkalimetallhydroxidlauge -, konnten die Ausbeute und/oder Raum-Zeit-Ausbeute an Isophorondiamin erhöht werden.

## Beschreibung

Die Erfindung richtet sich auf ein verbessertes Verfahren zur Herstellung von 3-Aminomethyl-3,5,5-trimethylcyclohexylamin, nachfolgend auch Isophorondiamin oder abgekürzt IPDA genannt, aus 3-Cyano-3,5,5-trimethylcyclohexanon, nachfolgend auch Isophoronnitril oder abgekürzt IPN genannt, umfassend eine erste Stufe zur mindestens teilweisen Überführung von Isophoronnitril in Isophoronnitrilimin und eine zweite Stufe zur aminierenden Hydrierung des Reaktionsgemischs der ersten Stufe mit Wasserstoff und Ammoniak in Gegenwart eines niederen Alkohols und eines Festbett-Hydrierkatalysators auf der Basis von Kobalt nach Raney. Das erfindungsgemäße Verfahren erlaubt die kontinuierliche Herstellung von Isophorondiamin in hoher Reinheit mit höherer Ausbeute und/oder höherer Raum-Zeit-Ausbeute als vorbekannte gattungsgemäße zweistufige Verfahren.

Isophorondiamin wird als Ausgangsprodukt zur Herstellung von Isophorondiisocyanat, einer Isocyanatkomponente für Polyurethansysteme, als Aminkomponente für Polyamide und als Härter für Epoxidharze verwendet. Isophorondiamin wird üblicherweise aus Isophoronnitril hergestellt, wobei in Gegenwart von Ammoniak, Wasserstoff und üblichen Hydrierkatalysatoren die Carbonylgruppe in eine Aminogruppe und die Nitrilgruppe in eine Aminomethylgruppe überführt werden. Das Ausgangsprodukt Isophoronnitril läßt sich in bekannter Weise durch Anlagerung von Cyanwasserstoff an Isophoron erhalten - siehe DE-OS 39 42 371.

Gemäß dem in der US 3,352,913 beschriebenen Verfahren zur Herstellung von Isophorondiamin aus Isophoronnitril erfolgt die Hydrierung in Gegenwart von Ammoniak und in Gegenwart von an sich bekannten kobalt-, nickel-, eisen- oder edelmetallhaltigen Katalysatoren bei 50 bis 150 °C und einem Druck von wenigstens 50 bar. Beispielsgemäß erfolgt die Hydrierung in Gegenwart von Methanol als Lösungsmittel unter Verwendung von Suspensions- oder Festbettkatalysatoren. Außer dem gewünschten Isophorondiamin entstehen in größerer Menge Nebenprodukte, wie insbesondere 3-Aminomethyl-3,5,5-trimethylcyclohexanol (Isophoronaminoalkohol, IPAA). Als nachteilig an diesem Verfahren erweisen sich die geringe Ausbeute sowie ein erheblicher Anteil an Nebenprodukten.

Im Bestreben, eine höhere Ausbeute an IPDA zu erhalten und den Zwangsanfall an IPAA zu minimieren, lehrt die DE-OS 30 11 656 ein zweistufiges Verfahren, wobei in der ersten Stufe IPN katalysatorfrei mit überschüssigem Ammoniak in 3-Cyano-3,5,5-trimethyl-iminocyclohexan überführt und dieses in der zweiten Stufe zu IPDA hydriert wird. Nachteilig an diesem Verfahren ist, daß außer dem eigentlichen Hydrierreaktor ein spezieller Iminbildungsreaktor erforderlich ist.

Gemäß der EP-B 0 042 119 wird eine weitere Verbesserung des Verfahrens darin gesehen, das Isophoronnitril vor der Reaktion desselben mit Ammoniak und Wasserstoff in Gegenwart von Hydrierkatalysatoren bei Temperaturen von 10 bis 120 °C und Drucken von 1 bis 300 bar einer Vorreaktion mit Ammoniak in Gegenwart von anorganischen und organischen Ionenaustauschern in der Ammoniumform als Iminbildungskatalysatoren zu unterwerfen. Während das Volumenverhältnis Isophoronnitril zu Ammoniak in der Iminbildungsstufe 1 zu 0,5 bis 20 betragen soll, wird dieses Verhältnis in der Hydrierstufe auf 1 zu 10 bis 20 erhöht. Das Verfahren, das sich in Rieselbettreaktoren ausführen läßt, führt zwar zu einer hohen Ausbeute an IPDA sowie zu einer hohen Reinheit, die Wirtschaftlichkeit des Verfahrens wird aber durch den hohen Ammoniaküberschuß, der einen sehr hohen Druck und damit eine aufwendige Hydrierapparatur erforderlich macht, beeinträchtigt.

Gemäß DE-A 44 26 472 läßt sich Isophoronnitril auch unter Verwendung eines trägergebundenen Heteropolysäure-Katalysators mit Ammoniak in das Isophoronnitrilamin und letzteres unter Verwendung üblicher Festbett-Hydrierkatalysatoren, darunter auch Raney®-Kobalt, in Isophorondiamin überführen. Nachteilig sind die in der Praxis erforderlichen hohen Hydrierdrucke - beispielgemäß 238 bar -, was einen hohen apparativen Aufwand erforderlich macht.

In der EP-B 0 042 119 wird auch ein Vergleichsbeispiel offenbart, wobei Isophoronnitril und flüssiges Ammoniak in einen mit handelsüblichem Kobaltkatalysator beschickten Festbett-Hydrierreaktor von oben hineingepumpt werden. Das Reaktionssystem wird mit H₂ auf 270 bar gehalten. Bei dieser einstufigen Ausführungsform werden trotz etwa quantitativer Umsetzung des Isophoronnitrils nach destillativer Aufarbeitung nur 48 % Isophorondiamin und daneben viele Nebenprodukte erhalten. Gemäß DE-A 43 43 890 konnte durch Verwendung eines C₁- bis C₃-Alkohols als Lösungsmittel der Druck auf 3 bis 10 MPa abgesenkt werden. Unter Rieselbettfahrweise konnte bei einem LHSV-Wert von 1 h⁻¹ (liquid hourly space velocity) und ohne Recyclierung von rückspaltbaren hochsiedenden Nebenprodukten eine Ausbeute von knapp 90 % erzielt werden.

Aus der EP-A 0 449 089 ist ein weiteres zweistufiges Verfahren zur Herstellung von Isophorondiamin bekannt: In zwei räumlich voneinander getrennten Reaktionsräumen wird eine Lösung von Isophoronnitril in Tetrahydrofuran zunächst mit überschüssigem Ammoniak an aciden Metallkatalysatoren umgesetzt und das Reaktionsgemisch in einem zweiten Reaktionsraum mit Wasserstoff in Gegenwart von überschüssigem Ammoniak an kobalt-, nickel-, ruthenium- und/oder anderen edelmetallhaltigen Katalysatoren und gegebenenfalls basischen Komponenten bei sehr hohem Druck hydriert. Der hohe Druck macht eine sehr aufwendige Hydrierapparatur erforderlich.

Die EP-A 0 394 967 umfaßt auch die Herstellung von Isophorondiamin aus Isophoronnitril, wobei IPN unter Bedingungen der reduktiven Aminierung in Gegenwart eines ersten Hydrierkatalysators bei mäßigen Temperaturen zunächst in das Aminonitril überführt und anschließend die Nitrilgruppe in Gegenwart eines gegenüber Nitrilgruppen hydrierwirksamen zweiten Hydrierkatalysators bei höhrerer Temperatur in eine Aminomethylgruppe überführt. Obgleich dieses Verfahren bei niedrigen Drucken durchgeführt werden kann, wird als wesentlicher Nachteil angesehen, daß ein streng einzuhaltendes Temperaturprogramm während der beiden Reaktionsstufen gefahren werden muß, wodurch die Raum-Zeit-Ausbeute und damit die Wirtschaftlichkeit des Verfahrens sinken. Sofern nicht spezielle Promotoren zusätzlich zum Einsatz kommen, entspricht die IPDA-Produktqualität wegen des meist zu hohen Gehalts an destillativ nicht abtrennbarem 3-Cyano-3,5,5-trimethyl-aminocyclohexan nicht den Anforderungen.

Im Verfahren gemäß JP-A 06-321870 wird ein in Abwesenheit eines Iminierungskatalysators, aber in Gegenwart von Methanol und Ammoniak aus Isophoronnitril erhaltenes Gleichgewichtsgemisch aus Isophoronnitril und Isophoronnitrilimin in Gegenwart eines Festbettkatalysators, enthaltend ein Katalysatormetall aus der Reihe Co, Ni, Ru und Pd, vorzugsweise Co auf Diatomit, aminierend hydriert. Die Ausbeute an IPDA liegt bei nur 88 %, und zudem ist die Raum-Zeit-Ausbeute niedrig. Gemäß JP-A 07-188126 wird im vorgenannten Verfahren zur aminierenden Hydrierung ein Festbettkatalysator auf der Basis von Raney-Kobalt eingesetzt, wobei der Katalysator erhalten wird durch Schmelzeliminierung von Aluminium aus einer binären oder ternären Legierung nach Raney. Auch hier beträgt die in den Beispielen angegebene Ausbeute an IPDA nur etwa 88 %.

Aufgabe der vorliegenden Erfindung ist, ein weiteres zweistufiges Verfahren zur Herstellung von IPDA und IPN aufzuzeigen, das in zuverlässiger Weise zu einer höheren Ausbeute an IPDA und/oder höheren Raum-Zeit-Ausbeute führt. IPDA sollte ferner in hoher Produktreinheit erhältlich sein. Die Hydrierstufe sollte ferner bei einem Druck von maximal 10 MPa, vorzugsweise unter 8 MPa, durchführbar sein, um den apparativen Aufwand möglichst gering zu halten.

Die Aufgabe wird gelöst durch ein Verfahren zur Herstellung von 3-Aminomethyl-3,5,5-trimethylcyclohexylamin (Isophorondiamin, IPDA) aus 3-Cyano-3,5,5-trimethylcyclohexanon (Isophoronnitril, IPN), umfassend eine erste Stufe,
wobei Isophoronnitril in An- oder Abwesenheit eines Iminierungskatalysators mit Ammoniak zumindest teilweise in 3-Cyano-3,5,5-trimethylcyclohexanimin überführt wird, und eine zweite Stufe,
wobei das Reaktionsgemisch der ersten Stufe, soweit noch nicht anwesend, nach Zugabe eines Alkohols mit 1 bis 3 C-Atomen, unter Verwendung eines Festbett-Hydrierkatalysators auf der Basis von Kobalt nach Raney bei einer Temperatur im Bereich von 50 bis 150 °C und einem Druck im Bereich von 3 bis 10 MPa mit Wasserstoff aminierend hydriert wird, das dadurch gekennzeichnet ist, daß der zu verwendende Hydrierkatalysator durch ein Verfahren hergestellt wurde, umfassend die Schritte: (i) inniges Mischen wenigstens einer pulverförmigen Kobaltlegierung und pulverförmigen Kobalts als Bindemittel, wobei die Kobaltlegierung Kobalt und gegebenenfalls Promotoren sowie eine auslaugbare Legierungskomponente aus der Reihe Aluminium, Zink und Silicium und das Pulvergemisch Kobalt und auslaugbare Legierungskomponenten im Gewichtsverhältnis zwischen 30 zu 70 und 75 zu 25 enthält, (ii) Sintern des Pulvergemischs zu einem mechanisch stabilen Formkörper mit einer Dichte von 1,3 bis 5,5 g/cm³, einem Porenvolumen bis 0,5 cm³/g (Wasseradsorption) sowie einer BET-Oberfläche (DIN 66 132) von unter 1 m²/g und (iii) Aktivieren des gesinterten Formkörpers durch zumindest teilweises Auslaugen der auslaugbaren Legierungskomponenten mittels einer Alkalimetallhydroxidlauge.

Der erfindungsgemäß zu verwendende Festbettkatalysator für die aminierende Hydrierung des aus der ersten Stufe erhaltenen Isophoronnitrilimin und Isophoronnitril enthaltenden Reaktionsgemischs ist erhältlich nach dem in der DE-A 43 35 360 und DE-A 43 45 265 beschriebenen Verfahren. Bezüglich der Auswahl und Stoffdaten der zur Herstellung zu verwendenden Rohstoffe - Katalysatormetall, Legierung und, sofern erwünscht, Promotoren sowie Hilfsstoffe, wie Verformungshilfsmittel, Gleitmittel, Plastifizierer und/oder Porenbildner - und Stoffdaten der Katalysatorvorstufe sowie der Einzelheiten der Verfahrensstufen, nämlich Mischen der Bestandteile, Sintern des Pulvergemischs zu Formkörpern der Katalysatorvorstufe und Aktivieren des Katalysators durch zumindest teilweises Auslaugen des auslaugbaren Legierungsbestandteils aus der Katalysatorvorstufe wird auf die genannten Dokumente verwiesen.

Als Einsatzgebiet für die in den zuvor genannten Dokumenten (DE-A 43 35 360 und DE-A 43 45 265) beschriebenen Katalysatoren werden die Hydrierung von Nitrogruppen, C-C-Doppelbindungen, Zuckern und aromatischen Ringen genannt. Hinweise oder eine Anregung, mit diesen Katalysatoren auch Imine und Nitrilgruppen aminierend zu hydrieren, lassen sich den genannten Dokumenten nicht entnehmen. Es war überraschend, daß die Verwendung dieser speziellen Festbettkatalysatoren auf der Basis von Kobalt nach Raney im gattungsgemäßen zweistufigen Verfahren zur Herstellung von IPDA aus IPN zu einer höheren Ausbeute bei meist sogar gleichzeitig höherer Raum-Zeit-Ausbeute führt. Es wurde ferner gefunden, daß entsprechende Katalysatoren auf der Basis von Nickel nach Raney bei der zweistufigen Herstellung von IPDA aus IPN deutlich weniger wirksam sind als solche auf der Basis von Kobalt. Auch gegenüber im gattungsgemäßen Verfahren zuvor eingesetzten Raney-Kobalt-Festbettkatalysatoren zeigten die erfindungsgemäß zu verwendenden Katalysatoren in der zweiten Stufe eine wesentlich höhere Katalysatorwirksamkeit.

In der ersten Stufe des erfindungsgemäßen Verfahrens wird zumindest ein Teil, vorzugsweise der größte Teil, des eingeführten Isophoronnitrils in Isophoronnitrilimin überführt. Das die Iminierungsstufe verlassende Reaktionsgemisch sollte Isophoronnitrilimin und Isophoronnitril möglichst im Molverhältnis von größer 1 enthalten. Zweckmäßigerweise wird die Iminierung des Isophoronnitrils bis zu einem Umsatz von über 80 %, vorzugsweise über 90 %, geführt.

Sofern die Iminierung in Abwesenheit eines Iminierungskatalysators durchgeführt wird, sind bei einer Reaktionstemperatur im Bereich zwischen 10 und etwa 60 °C mehrere Stunden erforderlich, um den gewünschten Iminierungsumsatz zu erzielen; zwar kann die Iminierung in Abwesenheit eines Katalysators auch bei höherer Temperatur als 60 °C, etwa bei 100 °C, durchgeführt werden, jedoch besteht hierbei die Gefahr einer erhöhten Nebenproduktbildung, wodurch die Produktreinheit des aus dem Reaktionsgemisch der zweiten Stufe nach destillativer Aufarbeitung erhaltene Isophorondiamins negativ beeinflußt wird. Um die Gleichgewichtseinstellung in der ersten Stufe zu beschleunigen, ist es zweckmäßig, einen Iminierungskatalysator zu verwenden. Hierzu können die aus dem Stand der Technik bekannten Iminierungskatalysatoren verwendet werden. Geeignete Iminierungskatalysatoren sind saure anorganische und organische Ionenaustauscher (siehe EP-B 0 042 119), acide Metalloxide, wie insbesondere Aluminiumoxid und Titandioxid (Anatas) (siehe EP-A 0 449 089), trägergebundene Heteropolysäuren (siehe DE-A 44 26 472) und saure Zeolithe. Bei Verwendung eines Iminierungskatalysators kann die Reaktionstemperatur im Bereich zwischen 10 und 150 °C, vorzugsweise zwischen 60 und 130 °C und insbesondere zwischen 80 und 120 °C, liegen.

Obgleich die Iminierung von Isophoronnitril mit flüssigem Ammoniak in Abwesenheit eines weiteren Lösungsmittels möglich ist, hat es sich als vorteilhaft herausgestellt, zusätzlich ein Lösungsmittel aus der Reihe eines Alkohols mit 1 bis 3 C-Atomen, vorzugsweise eines einwertigen primären Alkohols und insbesondere Methanol, zu verwenden. Vorzugsweise wird dem Iminierungsreaktor ein Isophoronnitril, flüssiges Ammoniak und Methanol enthaltendes Gemisch zugeführt. Das Gemisch enthält zweckmäßigerweise 10 bis 40 Gew.-%, vorzugsweise 10 bis 30 Gew.-%, Isophoronnitril und 10 bis 40 Gew.-%, vorzugsweise 20 bis 40 Gew.-%, Ammoniak. Es ist vorteilhaft, Isophoronnitril, Ammoniak und den Alkohol in einem solchen Verhältnis miteinander zu mischen, daß eine im wesentlichen homogene Lösung resultiert. Im Prinzip können die zuvor genannten Grenzwerte für Ammoniak und Isophoronnitril auch unter- oder überschritten werden, sofern hierbei eine im wesentlichen homogene Lösung resultiert.

Bei der Iminierung in Gegenwart eines Iminierungskatalysators kann der Katalysator in Form eines Suspensionskatalysators oder eines Festbettkatalysators zur Anwendung gelangen. Vorteilhaft ist die Verwendung eines Festbettkatalysators, da sich hierbei aufwendige Schritte zur Abtrennung des Reaktionsgemischs vom Katalysator erübrigen. Bei der Iminierung von Isophoronnitril in Gegenwart eines Festbettkatalysators wird dieser in Form üblicher Katalysatorformlinge, wie Strangpreßlinge, Pellets und Tabletten, als Schüttung in einem Festbettreaktor eingesetzt. Der Iminierungskatalysator kann in einem eigenen Reaktor angeordnet sein. Es ist jedoch auch möglich, den Iminierungskatalysator in einem Reaktor anzuordnen, der sowohl eine Schüttung des Iminierungskatalysators als auch eine Schüttung des für die aminierende Hydrierung eingesetzten Katalysators enthält. Je nachdem, ob der Reaktor als Rieselbettreaktor oder als Blasenreaktor betrieben wird, befindet sich die Schüttung des Iminierungskatalysators oberhalb (Rieselbettreaktor) beziehungsweise unterhalb (Blasenreaktor) der Schüttung des Hydrierkatalysators. Es hat sich als vorteilhaft erwiesen, einen einzigen Reaktor zu verwenden, der eine Schüttung des Hydrierkatalysators als auch eine Schüttung des Iminierungskatalysators enthält; vorzugsweise wird ein solcher Reaktor in Form eines Rieselbettreaktors betrieben. Hierbei wird das Gemisch aus Isophoronnitril, flüssigem Ammoniak und Alkohol, insbesondere Methanol, am Reaktorkopf aufgegeben. In diesen Fällen strömt zweckmäßigerweise gleichzeitig Wasserstoff für die aminierende Hydrierung von oben in den Reaktor.

Außer den zuvor genannten Bestandteilen des der Iminierungsstufe zuzuführenden Gemischs kann dieses zusätzlich höher oder tiefer als Isophorondiamin siedende Fraktionen aus der destillativen Aufarbeitung des aus dem Rieselbettreaktor abgezogenen Reaktionsgemischs enthalten. Derartige Fraktionen können außer Resten an Isophorondiamin auch solche Nebenprodukte enthalten, aus welchen sich unter den Reaktionsbedingungen erneut Isophorondiamin bildet. Durch Rückführung derartiger Fraktionen in das einzusetzende Gemisch läßt sich die Ausbeute an Isophorondiamin deutlich steigern. Besonders vorteilhaft ist es, die oberhalb Isophorondiamin siedende Fraktion, welche außer Resten an Isophorondiamin 3,3,5-Trimethyl-6-imino-7-aza-bicyclo-[3,2-1]-octan als Hauptprodukt enthält, zusammen mit dem Gemisch aus Isophoronnitril, Ammoniak und Lösungsmittel dem Rieselbettreaktor zuzuführen. Durch die Rückführung der das vorgenannte Nebenprodukt - eine bicyclische Verbindung mit Amidinstruktur - enthaltenden Fraktion ist es möglich, die Ausbeute an Isophorondiamin nennenswert zu steigern und damit die Wirtschaftlichkeit des Verfahrens zu erhöhen. Die das bicyclische Amidin enthaltende Fraktion kann, falls dies gewünscht ist, auch unmittelbar dem der zweiten Stufe zuzuführenden Reaktionsgemisch zugesetzt werden.

Bei dem in der zweiten Stufe des erfindungsgemäßen Verfahrens eingesetzten Katalysator handelt es sich um einen Festbettkatalysator. Zur Durchführung dieser Verfahrensstufe werden übliche Festbettreaktoren eingesetzt. Wie bereits bei der Iminierungsstufe abgehandelt, kann der Reaktor sowohl als Rieselbettreaktor als auch als Blasensäule betrieben werden, wobei jedoch, wie dies auch aus der DE-A 43 43 390 hervorgeht, die Rieselbettfahrweise bevorzugt ist.

Die Bauarten der Reaktoren sind dem Fachmann bekannt: In einem Behälter ist der Festbettkatalysator in Form einer oder mehrerer Schichten angeordnet; der Reaktor verfügt ferner über Vorrichtungen zum Temperieren der Katalysatorschichten, um die Aufrechterhaltung der gewünschten Temperatur in der jeweiligen Katalysatorschicht zu gewährleisten. Anstelle eines einzigen Rieselbettreaktors können auch mehrere Rieselbettreaktoren hintereinander geschaltet werden, wobei das aus dem ersten Reaktor austretende Reaktionsgemisch am Kopf des zweiten Reaktors wieder aufgegeben wird. Der bzw. die Rieselbettreaktoren verfügen ferner über geeignete Vorrichtungen zum Aufgeben des Reaktionsgemischs sowie des Wasserstoffs, ferner Vorrichtungen zum Verteilen der Flüssigkeit auf der Oberfläche des ersten Katalysatorbetts und schließlich geeignete Austragsvorrichtungen für das den Reaktor verlassende Reaktionsgemisch.

Die aminierende Hydrierung, also die zweite Reaktionsstufe, wird bei einer Temperatur im Bereich von 80 bis 150 °C, vorzugsweise 90 bis 130 °C, durchgeführt. Die aminierende Hydrierung erfolgt bei einem Druck im Bereich von 3 bis 10 MPa, vorzugsweise bei 5 bis 8 MPa und insbesondere unter 8 MPa. Durch die genannten mäßigen Betriebsdrucke, welche bei Verwendung der anspruchsgemäßen Gemische aus Isophoronnitril, Ammoniak und Lösungsmittel und der Rieselbettfahrweise unter den anspruchsgemäßen Temperaturbedingungen möglich sind, wird das Investitionsvolumen gemindert und damit die Wirtschaftlichkeit gegenüber Verfahren, welche einen hohen Betriebsdruck erfordern, erhöht. Unter dem angegebenen Druck wird der Gesamtdruck verstanden, der sich aus den Partialdrucken von Ammoniak, Wasserstoff, C₁- bis C₃-Alkohol sowie den übrigen Bestandteilen des Reaktionsgemischs zusammensetzt.

Das erforderliche Volumen an Festbettkatalysator für die zweite Stufe richtet sich nach dem vom Betriebsdruck, der Temperatur und Katalysatoraktivität abhängigen LHSV-Wert (liquid hourly space velocity), der eingehalten werden muß, um einen möglichst quantitativen Umsatz des Isophoronnitrilimin und Isophoronnitril enthaltenden Gemischs zu erzielen. Üblicherweise beträgt der LHSV-Wert mindestens 0,5 h⁻¹ und liegt vorzugsweise im Bereich über 0,5 h⁻¹ bis 3 h⁻¹. Es ist ein Vorteil des erfindungsgemäßen Verfahrens, daß bei einem LHSV-Wert um 2 h⁻¹, bei einer Reaktionstemperatur im Bereich zwischen 90 und 130 °C und einem Druck zwischen 5 und weniger als 8 MPa ein nahezu quantitativer Umsatz erzielt und IPDA in hoher Ausbeute und hoher Reinheit gewonnen werden kann. Der hohe LHSV-Wert führt zudem zu einer hohen Raum-Zeit-Ausbeute.

Bei der erfindungsgemäß besonders bevorzugten Ausführungsform, wonach ein Rieselbettreaktor eine untere Schüttung aus Hydrierkatalysator und eine obere Schüttung aus Iminierungskatalysator enthält, wird die jeweilige Schütthöhe der entsprechenden Katalysatoraktivität angepaßt - der Fachmann wird diese Aktivität und damit die Schütthöhe anhand von einfach durchzuführenden Vorversuchen ermitteln. Wie aus den Beispielen hervorgeht, reicht bei Verwendung von Titandioxid als Iminierungskatalysator ein Schüttvolumen aus, das etwa ein Viertel desjenigen des Hydrierkatalysators ausmacht.

Der für die Hydrierung erforderliche Wasserstoff kann dem Reaktor entweder im Überschuß zugeführt werden oder in einer solchen Menge, daß kein Wasserstoff aus dem Reaktor ausgetragen und rezykliert werden muß. Vorzugsweise wird Wasserstoff nicht im Überschuß zugeführt, um den technischen Aufwand zur Abtrennung dieses Überschusses, zur Kondensation des in ihm enthaltenen Ammoniaks und Lösungsmittels sowie zur Kompression des gereinigten Wasserstoffs und Rezyklierung zu vermeiden.

Das erfindungsgemäße Verfahren zeichnet sich durch seine Einfachheit, geringes Investitionsvolumen, hohe Ausbeute, hohe Raum-Zeit-Ausbeute und hohe IPDA-Produktreinheit aus.

### Beispiel 1

Ein als Rieselbettreaktor betriebenes Reaktionsrohr wurde im unteren Bereich mit 160 ml Hydrierkatalysator und im darüber liegenden Bereich mit 40 ml Iminierungskatalysator gefüllt. Als Iminierungskatalysator wurde Titandioxid (Titandioxid P 25 der Degussa AG) in Form von 1 mm Extrudaten eingesetzt. Als Hydrierkatalysator wurde aktivierter Kobaltkatalysator nach Raney, hergestellt gemäß DE-Patentanmeldung 43 45 265.5, in Form von Tabletten mit 5 mm Höhe und 3 mm Durchmesser eingesetzt. Die Einsatzlösung, die IPN und Methanol enthielt, sowie flüssiges Ammoniak wurden von oben in das Reaktionsrohr gepumpt. Auch der Wasserstoff wurde von oben in das Reaktionsrohr geleitet. Die Reaktionstemperatur wurde durch eine Ölheizung auf 100 °C gehalten. Der Druck wurde auf 6 MPa geregelt. Die Flüssigkeit wurde in einem Abscheidegefäß aufgefangen. Der Gasstrom am Reaktoreingang wurde so eingestellt, daß der gesamte Wasserstoff verbraucht wurde.

In der Einsatzlösung waren 24 Gew.-% IPN und 76 Gew.-% Methanol enthalten. 260 ml/h dieser Einsatzlösung und 140 ml/h Ammoniak wurden unmittelbar vor der Aufgabe auf den Reaktor gemischt und das Gemisch in den Reaktor gepumpt. Der LHSV-Wert betrug also 2 h⁻¹.

Nach der Analyse des Produktgemisches ergab sich eine Ausbeute von 92,2 % Isophorondiamin (=IPDA), bezogen auf eingesetztes IPN. Außerdem waren 3 % 2-Aza-4,6,6-trimethylbicyclo-3,2,1-octan (=Bicyklus) und 3,3 % 3,5,5-Trimethyl-6-imino-7-aza-bicyclo-3,2,1-octan (=Amidin) im Produktgemisch enthalten. Nach der Destillation des flüssigen Produktgemisches ergab sich eine Produktreinheit von 99,8 %. Methyl-IPDA war nur in einer Menge von 200 ppm nachweisbar.

Aus dem Vergleich mit den nachfolgenden Vergleichsbeispielen 1 und 2 folgt, daß die Verwendung des erfindungsgemäßen Hydrierkatalysators aufgrund dessen höherer Hydrieraktivität zu einer überraschend hohen Steigerung der Ausbeute und Raum-Zeit-Ausbeute führt.

### Vergleichsbeispiel 1

Ein als Rieselbett betriebenes Reaktionsrohr wurde im unteren Bereich mit 160 ml Hydrierkatalysator und im darüber liegenden Bereich mit 40 ml Iminierungskatalysator gefüllt: Während der Iminierungskatalysator demjenigen des Beispiels 1 entsprach (TiO₂ P 25 der Degussa AG), wurde als Hydrierkatalysator ein handelsüblicher Kobalt-Trägerkatalysator auf der Basis von 50 % Kobalt auf einem silikatischen Träger (Strangpreßlinge mit 4-5 mm Durchmesser und Höhe) eingesetzt. Die Betriebsbedingungen - Druck, Temperatur, LHSV-Wert, Mengenstrom und Konzentration der methanolischen IPN-Einsatzlösung und Mengenstrom des flüssigen Ammoniaks - entsprachen jenen des Beispiels 1.

Nach der Analyse des Produktgemischs ergab sich eine Ausbeute von 82 % IPDA.

### Vergleichsbespiel 2

Vergleichsbeispiel 1 wurde wiederholt, wobei jedoch als einziger Unterschied die Flüssigkeitsvolumenströme verändert wurden: 130 ml/h methanolische IPN-Einsatzlösung (24 Gew.-% IPN) und 70 ml/h flüssiges Ammoniak wurden unmittelbar vor der Aufgabe auf den Reaktor gemischt und das Gemisch in den Reaktor gepumpt. Der LHSV-Wert betrug also 1 h⁻¹.

Nach der Analyse des Produktgemisches ergab sich eine Ausbeute von 90,7 % IPDA, bezogen auf einsetztes IPN. Außerdem waren 4 % Bicyklus und 3,2 % Amidin im Produktgemisch enthalten. Die Destillation des Produktgemisches ergab eine Produktreinheit von 99,8 GC-Fl.-%.

Die Steigerung der Ausbeute, welche immer noch unter derjenigen des erfindungsgemäßen Beispiels 1 lag, wurde mit einer Halbierung der Raum-Zeit-Ausbeute erkauft.

### Vergleichsbeispiel 3

Die Umsetzung wurde analog Beispiel 1, jedoch unter Verwendung inerter Stahlkugeln von 2 mm Durchmesser anstelle des TiO₂-Iminierungskatalysators, durchgeführt. Alle übrigen Versuchsbedingungen entsprachen Beispiel 1.

Nach der Analyse des Produktgemisches ergab sich eine Ausbeute von 73,7 % IPDA.

Dieses Vergleichsbeispiel zeigt, daß in Abwesenheit eines wirksamen Iminierungskatalysators, jedoch unter Verwendung eines erfindungsgemäßen aktivierten Kobaltkataylsators nach Raney als Hydrierkatalysator, nur eine mäßige IPDA-Ausbeute erhalten wird.

### Beispiel 2

Es wurde eine 15 Gew.-% IPN, 30 Gew.-% Ammoniak und 55 Gew.-% Methanol enthaltende Lösung hergestellt und in einem Druckgefäß unter dem sich einstellenden Druck 24 h bei 25 °C gerührt; hierbei wurde Isophoronnitril (IPN) weitgehend in Isophoroniminonitril (IPIN) überführt. Diese Lösung wurde dann mit 400 ml/h, entsprechend einem LHSV-Wert von 2 h⁻¹, über den Reaktor gepumpt, der mit 160 ml erfindungsgemäßem Hydrierkatalysator (wie in Beispiel 1) und 40 ml inerten Stahlkugeln gefüllt war. Nach der Analyse des Produktgemisches ergab sich eine Ausbeute von 92,1 % IPDA. Nach der Destillation des Produktgemisches wurde eine Produktreinheit von 99,78 % IPDA erzielt.

### Beispiel 3

In einem gemäß Beispiel 1 befüllten Reaktionsrohr wurde die Umsetzung - Iminierung mit nachfolgender Hydrierung - analog Beispiel 1 durchgeführt, wobei als einziger Unterschied ein LHSV-Wert von 1 h⁻¹ eingestellt wurde. Nach der Analyse des Produktgemisches ergab sich eine Ausbeute von 94,2 % IPDA, bezogen auf eingesetztes IPN. Außerdem waren Bicyklus und 1 % Amidin im Produktgemisch enthalten.

Das Beispiel zeigt, daß durch eine Erniedrigung des LHSV-Wertes (im Vergleich zu Beispiel 1) die Ausbeute an IPDA noch deutlich gesteigert werden konnte.

## Patentansprüche

1. Verfahren zur Herstellung von 3-Aminomethyl-3,5,5-trimethylcyclohexylamin (Isophorondiamin, IPDA) aus 3-Cyano-3,5,5-trimethylcyclohexanon (Isophoronnitril, IPN),
umfassend eine erste Stufe,
wobei Isophoronnitril in An- oder Abwesenheit eines Iminierungskatalysators mit Ammoniak zumindest teilweise in 3-Cyano-3,5,5-trimethylcyclohexanimin überführt wird,
und eine zweite Stufe,
wobei das Reaktionsgemisch der ersten Stufe, soweit noch nicht anwesend, nach Zugabe eines Alkohols mit 1 bis 3 C-Atomen, unter Verwendung eines Festbett-Hydrierkatalysators auf der Basis von Kobalt nach Raney bei einer Temperatur im Bereich von 50 bis 150 °C und einem Druck im Bereich von 3 bis 10 MPa mit Wasserstoff aminierend hydriert wird,
dadurch gekennzeichnet,
daß der zu verwendende Hydrierkatalysator durch ein Verfahren hergestellt wurde, umfassend die Schritte: (i) inniges Mischen wenigstens einer pulverförmigen Kobaltlegierung und pulverförmigen Kobalts als Bindemittel, wobei die Kobaltlegierung Kobalt und gegebenenfalls Promotoren sowie eine auslaugbare Legierungskomponente aus der Reihe Aluminium, Zink und Silicium und das Pulvergemisch Kobalt und auslaugbare Legierungskomponenten im Gewichtsverhältnis zwischen 30 zu 70 und 75 zu 25 enthält, (ii) Sintern des Pulvergemischs zu einem mechanisch stabilen Formkörper mit einer Dichte von 1,3 bis 5,5 g/cm³, einem Porenvolumen bis 0,5 cm³/g (Wasseradsorption) sowie einer BET-Oberfläche (DIN 66 132) von unter 1 m²/g und (iii) Aktivieren des gesinterten Formkörpers durch zumindest teilweises Auslaugen der auslaugbaren Legierungskomponenten mittels einer Alkalimetallhydroxidlauge.

2. Verfahren nach Anspruch 1,
dadurch gekennzeichnet,
daß man in der ersten Stufe einen Iminierungskatalysator aus der Reihe saurer Oxide, Zeolithe, saurer Ionenaustauscher und trägergebundener Heteropolysäuren verwendet und die Iminierung in Gegenwart eines C₁- bis C₃-Alkohols durchführt.

3. Verfahren nach Anspruch 2,
dadurch gekennzeichnet,
daß man den Iminierungskatalysator in Form mechanisch stabiler Formkörper in einem Festbettreaktor einsetzt.

4. Verfahren nach einem der Ansprüche 1 bis 3,
dadurch gekennzeichnet,
daß der Festbett-Hydrierkatalysator in einem Hydrierreaktor angeordnet ist und man den Reaktor als Rieselbettreaktor betreibt.

5. Verfahren nach einem der Ansprüche 1 bis 4,
dadurch gekennzeichnet,
daß man in einem Festbettreaktor eine Schüttung eines Festbett-Iminierungskatalysators oberhalb einer Schüttung eines Festbett-Hydrierkatalysators anordnet und den Reaktor als Rieselbettreaktor betreibt.

6. Verfahren nach einem der Ansprüche 1 bis 5,
dadurch gekennzeichnet,
daß man die aminierende Hydrierung bei einer Temperatur im Bereich von 90 bis 130 °C und einem Druck im Bereich von 5 bis 8 MPa durchführt.

7. Verfahren nach einem der Ansprüche 1 bis 6,
dadurch gekennzeichnet,
daß man der Iminierungsstufe ein Gemisch aus im wesentlichen 10 bis 40 Gew.-% Isophoronnitril, 10 bis 40 Gew.-% Ammoniak und Methanol zuführt.

8. Verfahren nach einem der Ansprüche 1 bis 7,
dadurch gekennzeichnet,
daß man das Reaktionsgemisch aus der zweiten Stufe destillativ aufarbeitet und eine höher als Isophorondiamin siedende, 3,5,5-Trimethyl-6-imino-7-aza-bicyclo-[3,2,1]-octan (=Amidin) enthaltende Hochsiederfraktion zusammen mit einer Isophoronnitril, Ammoniak sowie Methanol enthaltenden Lösung der ersten Stufe zuführt.
